(19) Europäisches Patentamt / European Patent Office / Office européen des brevets

(11) EP 1 776 333 B1

(12) EUROPÄISCHE PATENTSCHRIFT

(45) Veröffentlichungstag und Bekanntmachung des Hinweises auf die Patenterteilung:
**12.01.2011 Patentblatt 2011/02**

(21) Anmeldenummer: **05771150.9**

(22) Anmeldetag: **20.07.2005**

(51) Int Cl.:
***C07C 211/63*** *(2006.01)*

(86) Internationale Anmeldenummer:
**PCT/DE2005/001288**

(87) Internationale Veröffentlichungsnummer:
**WO 2006/007835 (26.01.2006 Gazette 2006/04)**

# (54) AMMONIUMSALZE UND AMMONIUMSALZ-MINERALSALZCLATHRATE ALS TRANSPORT- UND WIRKFORM FÜR PHARMAZEUTISCH-MEDIZINISCHE UND ALS PHASENTRANSFERMITTEL FÜR CHEMISCHE ANWENDUNGEN

AMMONIUM SALTS AND AMMONIUM SALT/MINERAL SALT CLATHRATE COMPOUNDS FOR USE AS VEHICLE AND EFFECTIVE FORM FOR PHARMACO-MEDICAL APPLICATIONS AND FOR USE AS PHASE TRANSFER AGENTS FOR CHEMICAL APPLICATIONS

SELS D'AMMONIUM ET CLATHRATES DE SELS D'AMMONIUM ET DE SELS MINERAUX CONSTITUANT UNE FORME DE TRANSPORT ET UNE FORME ACTIVE POUR DES APPLICATIONS MEDICO-PHARMACEUTIQUES ET DES AGENTS DE TRANSFERT DE PHASE POUR DES APPLICATIONS CHIMIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.07.2004 DE 102004035808**

(43) Veröffentlichungstag der Anmeldung:
**25.04.2007 Patentblatt 2007/17**

(73) Patentinhaber: **Kasch, Helmut**
**07747 Jena (DE)**

(72) Erfinder:
- **REUTER, Uwe**
  **07973 Greiz (DE)**
- **OETTMEIER, Ralf**
  **07973 Greiz (DE)**
- **KASCH, Helmut**
  **07747 Jena (DE)**

(74) Vertreter: **Donath, Dirk**
**Patent- und Rechtsanwälte Bock Bieber Donath**
**Hans-Knöll-Strasse 1**
**07745 Jena (DE)**

(56) Entgegenhaltungen:

| | |
|---|---|
| **EP-A- 0 269 949** | **EP-A- 0 326 723** |
| **WO-A-92/22547** | **GB-A- 349 640** |
| **GB-A- 1 000 405** | **US-A- 2 873 282** |
| **US-A- 3 729 975** | **US-A- 4 921 854** |
| **US-A- 5 149 320** | **US-A- 5 169 942** |
| **US-A- 5 504 922** | **US-B1- 6 194 456** |

- **C. VENTURELLO, R. D'ALOISIO: "A convenient syntesis of 1,2-alkanediyl carbonates" SYNTHESIS, 1985, Seiten 33-34, XP002360168**
- **A. INESI, V. MUCCIANTE, L. ROSSI: "A convenient method for the synthesis of carbamate esters from amines and tetraethylammonium hydrogen carbonate" JOURNAL OF ORGANIC CHEMISTRY, Bd. 63, 1998, Seiten 1337-1338, XP002360169**
- **PATENT ABSTRACTS OF JAPAN Bd. 017, Nr. 185 (C-1047), 12. April 1993 (1993-04-12) & JP 04 338332 A (TAKEDA CHEM IND LTD), 25. November 1992 (1992-11-25)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 776 333 B1

**Beschreibung**

Die Erfindung betrifft stabile lagerungsfähige Ammonium / Mineralsalz-Clathrate (Cluster, Einschlussverbindungen) mit sauren zweibasischen Säureresten wie Hydrogencarbonat, Verfahren zu deren Herstellung sowie pharmazeutisch-medizinische und chemisch synthetische Anwendungen für diese Verbindungen.

Im Vordergrund der pharmazeutisch-medizinischen Anwendungen steht die Nutzung der Ammoniumsalze in Form ihrer sauren Salze und stabilen Salzcluster als so genannte Prodrugs in Verbindung mit dem integrierten Wirkstöffmolekül, während bei den chemisch synthetischen Anwendungen die Nutzung der Ammoniumsalze als Phasentransferkatalysatoren für die enantioselektive bzw. diastereoselektive Synthese von Wirk- und Wertstoffen wie cyclischen Carbonaten via Halogenhydrine im Fokus steht.

Ein Nachteil vieler Wirkstoffe besteht bei pharmazeutisch-medizinischen Anwendungen oft darin, dass sie trotz Wirksamkeit *in vitro* bei den meisten Applikationsrouten ihr eigentliches Zielorgan gar nicht erreichen, weil sie auf dem Transport dorthin metabolisiert und unwirksam geworden sind. Um bei einem Wirkstoff diese Veränderungen zu vermeiden, überführt man sie in der Regel in solche stabilen Produkte, die nach Möglichkeit erst in der Zelle den eigentlichen Wirkstoff oder aber wie z.B. bei der Leberpassage unter dem Einfluss diverser Enzyme den Wirkstoff freisetzen. Dabei ist es wichtig, den Wirkstoff kontrolliert freizusetzen und eine hohe Bioverfügbarkeit zu garantieren. Viele Wirkstoffe, z. B. bei systemischen Anwendungen, werden durch parenterale Gabe letztendlich über die Blutbahn zu den Zielorganen befördert. Der Wirkstoff muss dem ihn umgebenden Medium angepasst sein. Bei Infusionen, besonders aber bei subcutaner und oraler Gabe kann es zu einer Abnahme der Wirksamkeit bis hin zum totalen Ausfall kommen. Letzteres wird vor allem bei oraler Gabe bei der Magenpassage häufig beobachtet. Bei den als Lokalanästhetika bekannten basischen Estern vom Procaintyp weiß man, dass diese insbesondere durch Esterasen wie Cholinesterasen gespalten werden. In der Literatur sind bisher keine Prodrugs für Procain und vergleichbare Produkte bekannt, die zu einer adequaten Wirkung und Bioverfügbarkeit geführt haben, wie sie bei Infusionen z.B mit Procainhydrochlorid und Natriumbicarbonat (Weber, Oettmeler, Reuter: PCT/EP 98/01742 und US 6,194,456 B1; Dhaliwal, Masih US 5149320; US 5209724, Shumakov, Onishchenko et.al. SU 878297; Thut, Turner US 5505922) oder einer aus Procainhydrochlorid, Natriumcarbonat oder -hydrogencarbonat und $CO_2$ frisch bereiteten Infusionslösung (Abel & Imray GB 349 640 A) beobachtet wurden. Bekannte Präparate wie Novacain, bei dem eine hämolytische Wirkung nachgewiesen wurde (E.R. Hammerland u. K. Pederson-Bjergard; J. pharm. Sci 1961, 50, 24) und das bei der basenkatalytischen Hydrolyse entstehende basische und beschränkt lösliche Diprocainiumcarbonat erfüllen diese Kriterien ebenso nicht wie Procain-Wirkstoffkonjugate (Kasch, Goldschmidtt: PCT/EP 00/13036). Die Nutzung von so genannten Modifiern (PCT/US 93/05631) hat hierbei bisher nicht zum Erfolg geführt. Procain und seine Analoga besitzen eine vielfältige biologische Wirkung, die bei parenteraler Gabe aber nicht ausreichend genutzt werden kann, weil diese Pharmaka schlecht resorbiert werden und somit schlecht bioverfügbar sind. Das liegt unter anderem daran, dass deren Löslichkeit beschränkt und diese die Tendenz zeigen, gefällt zu werden. Hinzu kommt, dass frisch bereitete Infusionslösungen z.B. in Kombination mit Basen nur begrenzt haltbar sind und schon binnen 30 Minuten bei Temperaturen > 30°C metabolisiert werden, wobei eine Spaltung in p-Aminobenzoesäure und Diethylaminoethanol erfolgt. Um einer Zersetzung von Procainanaloga, -insbesondere Procainhydrochlorid entgegenzutreten, werden Stabilisatoren wie Benzylalkohol zugesetzt, die ihrerseits aber zur Auslösung, ungewollter Nebenwirkungen, z.B. Allergien, führen können. Eine weitere Ursache besteht in vielen Fällen in einer unausgewogenen und gestörten Isotonie und / oder Isohydrie.

Bei chemisch synthetischen Anwendungen, z.B. bei der Herstellung cyclischer Carbonate aus Halogenhydrinen, die für Wirk- und Wertstoffe, z.B. für PET (Positroronen-Emissions-Tomographie), benötigt werden, bediente man sich bisher aufwendiger, umweltbelastender und wenig effektiver Verfahren (Umsetzung mit Phosgen, Urethanherstellung, Umlagerungsrektionen). Enantioselektive Synthesen oder diastereoselektive Synthesen wurden bisher nicht durchgeführt. Phasentransfersalze mit Hilfe derer aus vicinalen Halogenhydrinen cyclische Carbonate hergestellt werden, haben aufgrund mangelnder Ausbeuten bzw. Verwendung unpassender wässriger Lösungsmittel (C. Venturello, R. D. Aloisio, Synthesis 1985, S. 33-34; US 1,907,891) bisher keine technische Bedeutung erlangt. So konnten bei Verwendung von Tetrabutylammoniumhalogenid (Halogenid = $Cl^-$, $Br^-$ oder I) und $NaHCO_3$ nur geringfügige oder keine Umsätze festgestellt werden.

GB 349 640 A offenbart die Herstellung von therapeutischen Lösungen aus stickstoffhaltigen Basen enthaltend die Komponenten der Verbindung gemäß der Formel:

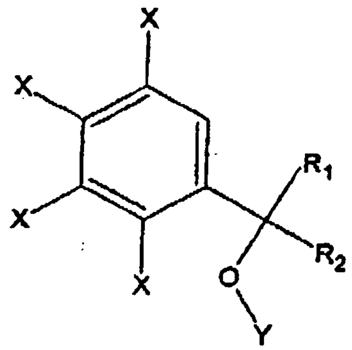

Das hierbei in Grundzügen erkannte Prinzip der Bereitung von Wirkstoff-Bicarbonat-Carbonat-Lösungen setzte sich nicht durch, daran änderte sich auch nichts mit der in US 6,194,456 offenbarten verbesserten Resorption und Löslichkeit, denn in der praktischen Umsetzung blieb es bei der auf parenterale Anwendungen beschränkten Applikation. Hinzu kamen schwerwiegende Komplikationen bei der Anwendung, was dazu führte, dass Pharmakologen und Arzneimittelzulassungsbehörden die kombination mit der Begründung ablehnten, dass weder ein definierter Wirkstoff, noch ein definierter Gehalt ersichtlich sind.

Der Erfindung liegt die Aufgabe zugrunde, Verbindungen und Verfahren zu deren Herstellung sowie pharmazeutisch-medizinische und chemisch synthetische Anwendungen anzugeben, die eine bessere Nutzung des Potentials von stickstoffhaltigen Basen, den eigentlichen Wirkformen für pharmazeutisch medizinische, Anwendungen, wie Procain, Lidocain oder Diethylaminoethanol einerseits ermöglichen und andererseits das chemisch synthetische Potential der Verbindungen auszuschöpfen, und damit die aus dem Stand der Technik bekannten Nachteile, vor allem die Sicherheitsbedenken bei der pharmazeutisch- medizinischen Anwendung, zu überwinden. Dabei sollen sowohl lipid- als auch wasserlösliche Darreichungsformen für die orale, demale und anale Applikation bereitgestellt werden.

Erfindungsgemäß wird dies durch Verbindungen für pharmazeutisch-medizinische und chemisch synthetische Anwendungen, umfassend Ammoniumsalz/Mineralsalzclathrate (Einschlussverbindungen, Cluster) mit sauren zweibasischen anionischen Säureresten der allgemeinen Formel I erreicht,

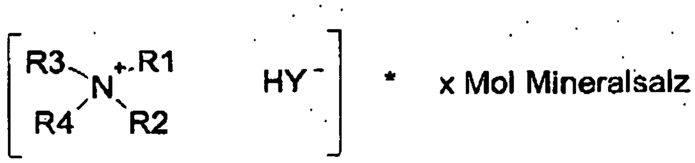

I

wobei R1, R2, R3 und R4 = Alkyl und substituiertes Alkyl geradkettig oder verzweigt, gegebenenfalls mit zusätzlicher Alkohol-, Ether-, Silytether-, Ester-, Amino- oder Amid-Funktion, H oder Aryl-alkyl, wobei unter Aryl ein aromatischer oder heteroaromatischer Ring mit gegebenenfalls zusätzlichen Substituenten, wie Alkyl mit 1 bis 4 C-Atomen, OH, $NR^*_2$ mit $R^*_2$= Alkyl mit Alkyl von 1 bis 4 C-Atomem oder H, COOH, COOR, CN, $NO_2$ zu verstehen ist und das kationische positivierte $N^+$ gegebenenfalls Bestandteil eines Wirkstoffes ist,

Y = einen zweibasischen Säurerest einer organischen Dicarbonsäure oder $CO_3^-$ , entsprechend $HY^-$ = $HCO_3^-$.

und x = 0,5 bis 30 die Zahl der Mineralsalzmoleküle für die clathratbildung darstellt,

Die erfindungsgemäßen Verbindungen können neben den zur Clathratbildung förderlichen Mineralsalzen Dextrane, Celluloseester oder Stärke, z.B. Maisstärke, als stabilisierende Stoffe enthalten.

Die erfindungsgemäßen Verbindungen können als für die Clathratbildung förderliche Mineralsalze ein-, zwei- und dreiwertige Metallsalzkationen wie $Na^+$, $K^+$, $Li^+$, $Mg^{++}$, $Ca^{++}$, $Zn^{++}$, $Fe^{++}$, $Fe^{+++}$, $Mn^{++}$ und als Anionen $Cl^-$, $Br^-$, $J^-$, $F^-$, $SO_4^-$, $SO_3^-$, $HSO_3^-$, $HCO_3^-$, $PO_4^{3-}$, $HPO_4^-$, $H_2PO_4^-$, $SiO_4^{4-}$, $AlO_2^-$, $SiO_3^-$und / oder $[(AlO_2)_{12}(SiO_2)_2]^{2-}$ enthalten.

Als erfindungsgemäße Verbindungen für pharmazeutisch-medizinische Zwecke werden Ammoniumsalz / Mineralsalzcluster, die von Basen als Wirkstoffkomponente abgeleitet sind, wobei unter Wirkstoffbasen Procain, substituierte Procaine, Epinephrin, Tetracain, Lidocain, Bupivacain, Pontocain, Propoxycain, Octacain, Mepivacain, Prilocain, Dibucain, Isocain, Marcain, Etidocain, Piridocain, Eucain, Butacain, Cocain, Articain, N,N-Diethylaminoethanol, N,N-Dimethylaminoethanol, N-Ethyl, N-methyl-aminoethanol oder N,N-Dietliylaminopropargyl mit freier und geschützter Alkoholfuntion, die verestert, verethert oder silyliert sein kann, für chemisch synthetische Anwendungen Ammoniumsalz / Mineralsalzcluster, die als Ammoniumsalzkomponente Tetraalkylammoniumhydrogencarbonate enthalten, zu verstehen sind.

Bevorzugte Verbindungen für pharmazeutisch-medizinische und chemisch synthetische Anwendungen aus den voran stehend aufgeführten erfindungsgemäßen Verbindungen sind:

Procainium- $[ProcH]^+$ (Fluor-,Chlor-, Brom- oder Iod-Procainium-) hydrogencarbonat
N-Alkyl-Procainium- $[Alkyl\text{-}Proc]^+$ hydrogencarbonat
Lidocainium-$[LidocainH]^+$ hydrogencarbonat,
N-Alkyl-Lidocainium-$[Alkyl\text{-}Lidocain]^+$ hydrogencarbonat .

Gegenstand der Erfindung ist weiterhin ein Verfahren zur Herstellung der erfindungsgemäßen Verbindungen, wobei erfindungsgemäß mineralsaure Ammoniumsalze, wie $NR_4HSO_4$, $NR_4HSO_3$, $(NR_4)_2HPO_4$, $NR_4H_2PO_4$, $NR_4$Halogenid, mit Halogenid = Cl, Br, I, und / oder organosaure Ammoniumsalze, wie $NR_4$Tosylat, $NR_4$OCO-(Akyl) -COOH, mit $R_4$ in der oben für R1, R2, R3 und R4 angegebenen Bedeutung und mit Alkyl = 0 bis 12 C-Atomen mit $NaHCO_3$, $NH_4HCO_3$, $Ca(HCO_3)_2$, $Mg(HCO_3)_2$ oder $KHCO_3$ in einem geeigneten Lösungsmittel, gegebenenfalls unter Zusatz von $CO_2$ auch in Form von Trockeneis unter Druck und für die Stabilisierung erforderlicher Salze, in die entsprechenden Mono-, Di-, Tri-substituierten oder quaternären Ammoniumhydrogencarbonate überführt werden.

Die erfindungsgemäßen Tetraalkylammoniumhydrogencarbonate wie Tetrabutylammoniumhydrogencarbonat oder N-Alkyl-Procainiumhydrogencarbonat werden vorzugsweise durch Umsetzung von $NR_4HSO_4$ mit $NaHCO_3$ oder $NH_4HCO_3$, wobei man Tetraalkylammoniumhydrogencarbonate für chemisch synthetische Anwendungen bevorzugt in situ in einem aprotonischen Lösungsmittel, z.B. Acetonitril, herstellt und als Reagenzien für Substrate, wie racemische und optisch aktive trans-1,2- bzw. trans-1,3- Halogenhydrine, mit Halogen = Cl, Br oder I, für die stereospezifische Herstellung cyclischer Carbonate direkt nutzt.

Die erfindungsgemäßen Ammoniumhydrogencarbonat / Mineraisalzclathrate (Einschlussverbindungen, Cluster) der oben angegebenen allgemeinen Formel I, $NR_4HCO_3$ x Mineralsalz, werden durch Umsetzung mineralsaurer oder zweibasischer organosaurer Ammoniumsalze in Gegenwart von $Metall^+$ und / oder $Metall^{++}$-hydrogencarbonaten, vorzugsweise Alkali-oder Erdalkali-Hydrogencarbonaten, und / oder Ammonium-hydrogencarbonat unter Zusatz von Kohlensäure ($H_2O$ / $CO_2$), die unter Druck dargestellt wird, und von gegebenenfalls weiteren Mineralsalzen und / oder Dextranen, Celluloseestern oder Stärke in der Kälte primär zur Umsetzung gebracht und anschließend durch wasserbindende Mittel, z.B. Mineralsalze, oder durch Gefriertrocknung entwässert werden, wobei aufgrund der stabilisierenden Wirkung der Mineralsalze in Form von Clathraten (EinschlussVerbindungen, Cluster) auch Dextranen, Celluloseestern oder Stärke diese als beständige lagerungsfähige Feststoffe für pharmazeutisch-medizinische und chemisch synthetische Anwendungen erhalten werden.

Mono-, Di-, Tri-Alkylammoniumhydrogencarbonate lassen sich erfindungsgemäß durch Umsetzung der basischen Amine, z.B. Procain, Lidocain oder Diethylaminoethanol, mit Ammoniumhydrogencarbonat ($NH_4HCO_3$) und / oder Kohlensäure, auch unter Zusatz von Trockeneis / Wasser, in der Kälte in situ herstellen, müssen aber zwecks Überführung in feste, stabile und lagerungsfähige Salze vor der Entwässerung mit einem Mineralsalz oder Dextran, Celluloseester oder Stärke, z.B. Maisstärke, als stabilisierenden zur Clathratbildung neigenden Stoff, versetzt werden.

Procainium-, Lidocainium- oder N,N-Diethyl,N-(1-hydroxyethyl)-ammonium-hydrogencarbonat enthaltende Mineralsalzclathrate (Cluster) lassen sich erfindungsgemäß durch Umsetzung von Procain, Lidocain bzw. Diethylaminoethanol mit Ammoniumhydrogencarbonat und stabilisierender Mineralsalze in der Kälte herstellen, wobei feste beständige Salzcluster nach Entwässerung erhalten werden.

Die erfindungsgemäßen Verbindungen können für pharmazeutisch medizinische Anwendungen zur Bekämpfung von Schmerzen und entzündlichen Prozessen, gegen Acidose, von Tumorerkrankungen, von Herz-Kreislauferkrankungen, von Autoimmunerkrankungen aufgrund verminderter Wirtsabwehr, zur Rekonvaleszenz und zur "wellness" , zur Stressptophyxlaxe sowie "antiageing" in der Geriatrie genutzt werden.

Die nach dem erfindungsgemäßen Verfahren herstellbaren Verbindungen können für pharmazeutisch-medizinische Anwendungen sowohl in fester Form für orale, dermale, nasale, anale und linguale oder in gelöster Form, auch in Suspension, für parenterale, peritoneale Gabe bzw. für Inhalationen genutzt werden, Zusätze weiterer Trägerstoffe, Stabilisatoren, Verdünnungsmitteln und sonstiger Hilfsmittel, wie sie auf dem Gebiet der Arzneimittel üblich sind, können enthalten sein, und nach Möglichkeit bei der Zubereitung unter Ausschluss protischer Lösungsmittel gearbeitet werden, extreme Erwärmung und Feuchtigkeit, außer bei kurzzeitigen Anwendungen, z.B. Infusionen, Injektionen oder Inhalationen, zu vermeiden sind.

Die nach dem erfindungsgemäßen Verfahren herstellbaren Verbindungen für pharmazeutisch-medizinische

Anwendungen können körpereigene Stoffe der Atmungskette, wie $CO_2$ und $HCO_3^-$ sowie überschüssiges Bicarbonat im Salzcluster für den Transport der Wirkstoffe nutzen, deren Bioverfügbarkeit durch zusätzliche Gabe von Carboanhydrasehemmem noch verbessert wird.

Die nach dem erfindungsgemäßen Verfahren herstellbaren festen Verbindungen, Salzcluster, die auch für die Bereitung von Infusions- und Injektionslösungen, für Tabletten oder Puder und Implantaten geeignet sind, verkörpern Wirkstoffcluster, die für ein besseres Targeting und eine verbesserte Bioverfügbarkeit aufgrund kontrollierter steuerbarer Freisetzung des Wirkstoffes beitragen.

Die nach dem erfindungsgemäßen Verfahren herstellbaren Verbindungen für pharmazeutisch-medizinische Anwendungen werden bei verbesserter Verträglichkeit und Bioverfügbarkeit in Mengen von 0,01 mg bis 2000 mg an effektivem Wirkstoff-eingesetzt.

Durch den Zusatz von Carboanhydrasehemmem wird der Transport und damit die Bioverfügbarkeit der Wirkstoffe verbessert. Dies beruht u.a. darauf, dass körpereigene Stoffe der Atmungskette, wie $CO_2$ und $HCO_3^-$ sowie überschüssiges Bicarbonat im Salzcluster für den Wirkstofftransport zusätzlich genutzt werden.

Nachfolgend noch einige Erläuterungen zu den wesentlichen Schritten, die zur überraschenden Überwindung der im Stand der Technik aufgeführten Probleme führte.

Es wurde überraschenderweise gefunden, dass substituierte Amine, primäre, sekundäre, tertiäre und quaternäre Amine, die auch als Bestandteil biologisch aktive Wirkstoffe fungieren können, auch saure Salze dieser, in Ammoniumsalze ($NR_4HCO_3$) mit Bicarbonat als Anion überführbar sind und als solche oder in Form von stabilen Clustern eine neue Qualität darstellen. Sie können aufgrund ihrer spezifischen Eigenschaften für neue medizinische und chemische Anwendungen genutzt werden. Diese Salze (Salzcluster) lassen sich entsprechend den nachfolgenden Bruttogleichungen herstellen:

a)

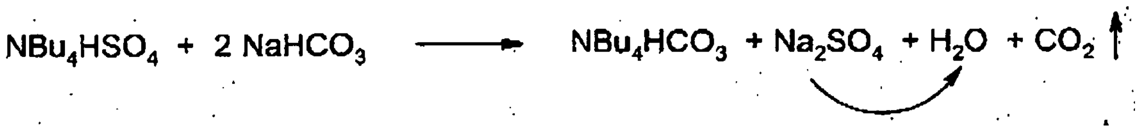

b) $NR_3H{+}HY{-} + 2\ NaHCO_3 \rightarrow NR_3H{+}HCO_3^- + Na_2Y + H_2O + CO_2\uparrow$

$NR_3H{+}Cl^- + NaHCO_3 \rightarrow NR_3H^+HCO_3 + NaCl$

c)

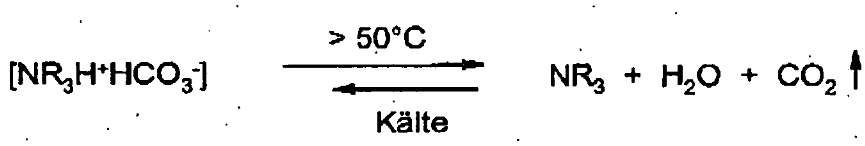


d)

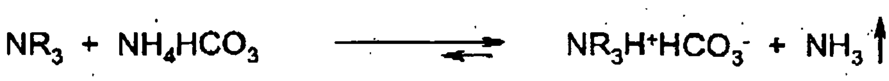

e)

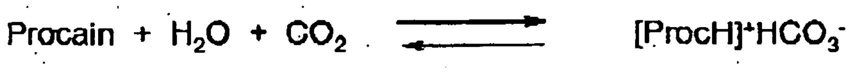

Die hier formulierten Umsetzungen im wasserfreien bzw. wasserhaltigen Milieu würden keine Besonderheit darstellen, wenn man entsprechend dem allgemeinen Stand der Technik nicht wüsste, dass Ammoniumhydrogencarbonate und dabei auch entsprechend N-substituierte Verbindungen als sehr unstabil gelten und ihre Herstellung in fester Form bisher in Frage gestellt wurde. Es wurde nun gefunden, dass sich N-substituierte Ammoniumhydrogencarbonate entsprechend obigen Umsatzgleichungen bilden. Dies lässt sich durch physikochemische analytische Daten wie der

Bestimmung der Leitfähigkeit und Gefrierpunkterniedrigung, massenspektrometrische Messungen, UV-und IR-Messungen sowie NMR-Untersuchungen in $D_2O$ belegen, die zur Strukturaufklärung genutzt wurden. Aus obigen Gleichungen geht aber auch hervor, und das lässt sich durch den Nachweis der entsprechenden Substanzen belegen, dass die Herstellung der festen beständigen und trockenen Substanzen in einer für medizinisch pharmazeutische und chemische Anwendungen erforderliche Qualität nicht ohne weiteres möglich ist, weil diese wieder in ihre Komponenten zerfallen können. Dies wird besonders dann beobachtet, wenn die spezifischen Reaktionsbedingungen für den Erhalt der Zielsubstanzen nicht peinlich eingehalten werdenl

$$[ProcH]^+HCO_3^- + Procain \xrightarrow[{[H_2O]}]{} [ProcH]_2\,CO_3\downarrow$$

$$[ProcH]_2\,CO_3 + H_2O + CO_2 \rightleftarrows 2\ ProcHHCO_3$$

Wenn es nicht gelingt, den Anteil an Procain gering oder auf Null zu halten und / oder gleichzeitig das Wasser vom Procainiumhydrogencarbonat fernzuhalten, wird sehr leicht Diprocainiumcarbonat gebildet, das in wässriger Lösung ausfällt. Aufgrund der erhöhten Basizität des Diprocainiumcarbonats wird auch die Verseifung des Procains initiiert. Um diesen Prozess bei der Herstellung der Ammoniumsalzcluster zu vermeiden oder umzukehren, wird Kohlensäure oder $CO_2$ , auch in Form von Trockeneis, und Wasser zugesetzt.

Eine erfindungsgemäße Lösung stellt ein Einbau der an und für sich instabilen Verbindungen wie Procainium- bzw. Lidocainiumhydrogencarbonat in Mineralsalze und / oder Dextrane, Stärke oder Cellulose zu so genannten Clathraten oder Clustern dar Dies gelingt überraschenderweise schon mit einfachen Mineralsalzen wie Natriumchlorid. In diesen Salzen, Clustern, sind die Ammoniumsalze entweder koordinativ in das Salzgitter integriert oder aber werden umhüllt und eingeschossen. Durch physikalisch chemische Modifizierung im Vergleich zu den bis Dato genutzten Infusionslösungen, die durch Zusammenmischen wässriger Lösungen von Procainhydrochlond und Natriumbicarbonat bei Raumtemperatur hergestellt wurden, konnte der Anteil an stark basischen Stoffen wie Procain und Carbonaten, z.B. Diprocainiumcarbonat, praktisch zurückgedrängt werden. Schon geringe Mengen an Carbonat katalysieren den Zerfall des Procainiumhydrogencarbonats in Procain oder Diprocainiumcarbonat. Durch das Herstellungsverfahren, das Absenken des pH durch Zusatz von $CO_2$ werden die Voraussetzungen geschaffen, den $NR_4HCO_3$-Gehalt auf praktisch 100 % zu erhöhen und durch den Einbau in Salze oder andere zur Clusterbildung befähigte Verbindungen auf eine originelle, biologischen Systemen adäquaten Weise, zu konservieren.

Die in den Salclustern vorhandenen Ammoniumhydrogencarbonate enthalten latent. Kohlensäure, die unter Normalbedingungen bei Raumtemperatur nicht, in wässriger Lösung und noch ausgeprägter in organisch-wässrigen Lösungen schnell freigesetzt werden.

Aus den Clathraten kann einerseits der Wirkstoff gezielt freigesetzt und für pharmazeutisch medizinische, andererseits das $CO_2$ bzw. das Hydrogencarbonat für chemisch synthetische Zwecke, z.B. als Reagenz zur stereoselektiven Herstellung cyclischer Carbonate, genutzt werden.

Kinetische Untersuchungen zum Metabolismus der Wirkstoffcluster weisen aus, dass die Stabilität der gelösten Verbindungen unter physiologischen Bedingungen ausreichend ist, um den Wirkstoff , z.B. Procain, systemisch aber auch lokal zu verabreichen und einen optimalen Transport zu den Targets zu garantieren. Der in den Clustern fixierte Wirkstoff, einschließlich Kohlensäure, bildet beim Auflösen in Wasser ein typisches Säure-Basen-Paar, das auch bei Gegenwart eines Überschusses an Bicarbonat eine hohe Pufferkapazität aufweist. Das Säure-Basen-Paar kann zu Aufrechterhaltung bzw. Korrektur des physiologischen pH, z.B. bei Acidose, genutzt werden und verfügt darüber hinaus über herausragende Eigenschaften. Dazu gehört die gute Löslichkeit, die eine Grundvoraussetzung für eine gute Bioverfügbarkeit darstellt. Das ausgewogene physiologische System des venösen Blutes einschließlich der $CO_2$/ $HCO_3^-$ -Balance wird durch die Salzcluster nicht beeinträchtigt. Das System ist sogar förderlich für die Stabilisierung des Säure-Basen-Paares.

Cholinesterasen, die durch Esterasehemmer , so auch Carboanhydrasehemmer gezielt gesteuert werden können, werden durch den Überschuss an Bicarbonat beeinträchtigt, was eine Verringerung der Enzymwirksamkeit nach sich zieht und indirekt die Spaltung und damit die Lebensdauer oder Verfügbarkeit von beispielsweise Procainiumsalz erhöht.

Die Existenz stabiler und wasserlöslicher N-substituierter Ammnoniumhydrogencarbonate vom Typ Procainiumhydrogencarbonat-Salzcluster in Verbindung mit zusätzlichem Bicarbonat in Form von beispielsweise Natriumbicarbonat ermöglicht einen patientenfreundlicheren Einsatz der Wirkstoffe und bleibt nicht auf Injektionen und Infusionen

beschränkt. Aufgrund einer definierten, analytisch exakt überprüfbaren Zusammensetzung der aus Salzcluster herstellbaren Lösungen ergibt sich auch hier eine Verbesserung im Vergleich zum Stand der Technik. Die physiologische Verträglichkeit sowie die Unbedenklichkeit der im angegebenen Dosisbereich einsetzbaren Salzcluster geht aus pH-Messungen und toxikologischen Untersuchungen hervor. In Abhängigkeit von dem zusätzlich eingesetzten Bicarbonat kann der pH-Wert der Infusionslösung durch das Säure-Base-Paar des Salzclusters innerhalb eines pH-Bereiches von 7,3 bis 8,3 eingestellt und konstant gehalten werden. Dies ermöglicht neben den schon aufgeführten Anwendungen den schonenden therapeutischen Einsatz bei Acidosen.

Die im Vergleich zum Procainhydrochlorid ausbleibende Hämolyse ist unter anderem auf die geringe Toxizität aufgrund der hohen Pufferkapazität des Salzclusters mit Procainiumhydrogencarbonat als Wirkstoff- und Carrierkomponente zurückzuführen. Untersuchungen mit dem Dunkelfeldmikroskop weisen aus, dass die Erythrocyten auch bei hohem Salzüberschuss in Takt bleiben und nicht zerplatzen.

Toxikologische Untersuchungen am Hühnerembryo zeigen einerseits die Herzwirksamkeit, die sich allerdings nur kurzzeitig in einer Erhöhung der Herzfrequenz bemerkbar macht und schnell wieder abklingt, nicht zuletzt aufgrund der dilatorischen Wirkung des Procainlumhydrogencarnonatsalzclusters, andererseits auch die gute Verträglichkeit. So wurden defekte Blutgefäße, die durch Verletzung, z.B. bei der Zugabe einer Ölsuspension der Salzcluster entstanden, unter Wirkstoffeinftuss repariert.

In Anwendungsuntersuchungen wurde das Wirkungspotential der Salzcluster überprüft, nachdem sichergestellt worden war, dass die Verträglichkeit noch besser gewährleistet ist, als das bei der Nutzung von Procainhydrochlorid der Fall ist. Durch die Nutzung der Salzcluster in Form eines Pulvers, als Kapseln oder Tabletten wird diese Schwelle noch weiter herabgesetzt. Eine Umhüllung der Tabletten wegen möglicher Zersetzung bei der Magen-Darm-Passage ist nicht unbedingt erforderlich, da sich beim Pressvorgang eine Schutzschicht bildet, die vorzugsweise im Darm aufgelöst wird.

Für nasale Anwendungen eignet sich einerseits das Pulver, das über Nasenspray mittels Treibgas verabreicht werden kann, andererseits ist eine Inhalation des in Kochsalz aufgelösten Pulvers (Wirkstoffgehalt 65 mg Procain / Inhalation) oder einer entsprechenden Tablette ein geeigneter Weg für eine lokale (geringfügig auch systemische) Anwendung im Nasen- und Nasenhöhlenbereich. Auf diese Weise können Schmerzen und Entzündungen in der Nasenhöhle therapiert und die Ausbreitung von Schmerzen auch auf benachbarte Ariale (Kopfschmerzen, Zahnschmerzen) vermieden werden. Diese Art Anwendung erfolgte bisher über Procain / Basen-Injektionen und lässt sich mit der Salzcluster-Lösung als Alternativa patientenfreuridlicher und optimaler durchführen.

Für die systemische Behandlung von Entzündungen, wie rheumatische Arthritis, Multiple Sklerose (MS), chronisch entzündliche Darmerkrankungen, Entzündungen der Nervenbahnen oder Entzündungen des Rückenmarks werden Corticoide mit allen ihren Nebenwirkungen eingesetzt. Procaincluster können auch hier bei systemischer oder lokaler Anwendung einen vergleichbaren antiinflammatorischen Effekt bei Langzeitbehandlung hervorrufen. Die unangenehmen Nebenwirkungen der Corticoide treten hierbei nicht auf.

Die prophylaktische Verabreichung von Procainclustern mindert die Folgen für die Ausbreitung bzw. Etablierung stressbedingter Erkrankungen, z.B. Tinnitus. Die Wirkung der Proccluster ist dabei unter anderem auf die neurogene und antioxidative Wirkung der Wirkstoffbasen zurückzuführen. Natriumbicarbonat im Überschuss stimuliert diesen Prozess, wie Untersuchungen an Macrophagen belegen (Chemilumineszenz an PMNL-Zellen).

Die Stabilisierung N-substituierter Ammoniumhydrogencarbonate durch Clusterbildung ermöglicht nicht nur die Herstellung dieser bisher als instabil eingestuften Verbindungen in fester Form, sie eröffnet aufgrund der vielfältigen Eigenschaften. z.B. als physiologisch angepasste Träger- und Transportform für den Wirkstoff eine wesentlich verbesserte Bioverfügbarkeit. Wässrige Lösungen können ohne belastende Zusätze aus den Clustern bzw. Clathraten für Injektionen und Infusionen bereitet werden. Ein weiterer Vorzug der Verbindungen ist deren chemischer Einsatz als Reagenz für die stereoselektive . Synthese von 1,3- und 1,2-cis (Z) cyclischen Carbonaten für PET (Positronen-Emissions-Tomographie).

Anhand der nachfolgenden Ausführungsbeispiele soll die Erfindung näher erläutert werden, ohne sie in irgendeiner Weise einzuschränken:

**I Zur chemisch synthetischen Nutzung von Tetraalkylammoniumhydrogen-carbonaten** (nicht erfindungsgemäß)

**Beispiel 1** 16β,17β-Carbonyldioxy-3-methoxy-estra-1,3,5(10)-trien

11 g 16α-Brom,17β-hydroxy-3-methoxy-estra-1,3,5(10)-trien (30,11 mMol) werden in 50 ml Acetonitril gelöst und nach Zugabe von 10 g $Bu_4NHSO_4$ und 20 g $NaHCO_3$ 16 Stunden bei Raumtemperatur gerührt. Das in situ erzeugte $Bu_4NHCO_3$ reagiert diastereoselektiv zum cis (Z)-cyclischen Carbonat, wobei das ebenfalls gebildete $Na_2SO_4$ dafür verantwortlich zeichnet, dass Wasserspuren gebunden werden. Nach erfolgter Umsetzung wird die Suspension filtriert, der Rückstand mit Acetonitril gewaschen und das Filtrat in ca. 100 ml fein zerstoßenes Eis eingerührt. Zur völligen Kristallisation lässt man im Kühlschrank ca. 16 Stunden stehen und erhält 8 g an 16β,17β-cyclischen Carbonat, welches

aus Methanol / Methylenchlorid umkristallisiert werden kann.
F.: 145 bis 150°C
IR [$cm^{-1}$]: 1496, 1604 (Aromat), 1788 (cycl. Carbonat)
MS [m/z]:$ES^-$ 341,5 (M-H; ber.f. M= 342,48)

**Beispiel 2** 16β,17β-Carbonyldioxy-3-methoxymethyloxy-estra-1,3,5(10)-trien

3 g 16α-Brom,17β-hydroxy-3-methoxy-estra-1,3,5(10)-trien (7,6 mMol) werden in 50 ml Acetonitril gelöst und nach Zugabe von 3 g $Bu_4NHSO_4$ und 6 g $NaHCO_3$ 16 Stunden bei Raumtemperatur gerührt. Nach erfolgter Umsetzung wird die Suspension filtriert, der Rückstand mit Acetonitril gewaschen und das Filtrat in ca. 50 ml fein zerstoßenes Eis eingerührt. Zur völligen Kristallisation lässt man im Kühlschrank ca. 16 Stunden stehen und erhält 2 g an 16β,17β-cyclischen Carbonat, welches aus Essigester umkristallisiert werden kann. Das acyclische Carbonat wird für die Herstellung von Prekursoren für PET (Positronen Emissions Tomographie) eingesetzt.
F.: 111 bis 115°C
IR [$cm^{-1}$]: 1496, 1604 (Aromat), 1790 (cycl. Carbonat)
MS [m/z]: $ES^-$ 357,5 (M-H; ber.f. M= 358,44)

**Beispiel 3** 16α,17α-Carbonyldioxy-3-methoxymethyloxy-estra-1,3,5(10)-trien

3 g 16β-Brom,17α-hydroxy-3-methoxymethytoxy-estra-1,3,5(10)-trien (7,6 mMol) werden analog Beispiel 2 zur Umsetzung gebracht. Nach Fällung in Eiswasser wird das 16α,17α-cyclische Carbonat abgefrittet und aus Essigester umkristallisiert.
IR [$cm^{-1}$]: 1496, 1604 (Aromat), 1789 (cycl. Carbonat)
MS [m/z]: $ES^-$ 357,5 (M-H; ber.f.M= 358,44)

**Beispiel 4** 16β,17β-Carbonyldioxy-3-methoxymethyloxy-5-androsten

3 g 16α-Brom,17β-hydroxy-3-methoxymethyloxy-5-androsten (7,25 mMol) werden in 50 ml Acetonitril gelöst und nach Zugabe von 3 g N-Ethyl-procainiumhydrogencarbonat [auch erzeugbar in situ aus N-Ethylprocainiumhydrogensulfat und $NaHCO_3$ oder N-Ethylprocainiumiodid, $NaHSO_4$, $NaHCO_3$) 30 Stunden bei Raumtemperatur gerührt. Nach erfolgter Umsetzung wird die Suspension fltriert, der Rückstand mit Acetonitril gewaschen und das Filtrat in ca. 50 ml Eiswasser eingerührt. Zwecks Isolierung des Steroids wird mit Ether extrahiert und der nach Abdampfen des Lösungsmittels verbleibende Rückstand an Kieselgel chromatographiert. Als Elutionsmittel wird ein Toluen / Essigester-Gemisch (30:1) verwendet. Man erhält 900mg des aus Essigester / Hexan kristallisierenden 16β,17β-cyclischen Carbonats.
F.: 144 bis 147°C
IR [$cm^{-1}$]: 1789 (cycl. Carbonat)
MS [m/z]: ES- 375,6 (M-H; ber. f. M= 376,5)

**II Zur pharmazeutisch-medizinischen Nutzung**

**Beispiel 5** Procainiumhydrogencarbonat-Salzcluster

<u>a) Proc*HCl ($NaHCO_3$ $H_2CO_3$ unterDruck)</u>

5,456 g Procainhydrochlorid (20 mMol) werden bei 0°C bis -4° C mit 100 mt einer gekühlten mit $CO_2$ unter Druck gesättigten wässrigen Lösung versetzt und anschließen 6,721 g $NaHCO_3$ zugegeben. Anschließend wird die klare homogene Lösung eingefroren und gefriergetrocknet. Die Gefriertrocknung erfolgt bis zur Gewichtskonstanz, d.h. bis keine Gewichtsabnahme mehr feststellbar ist. Es werden 11,9 g (97,7 % d.Th.) des Procainiumhydrogencarbonat enthaltenden Salzclusters erhalten, welches direkt für pharmazeutisch-medizinische und chemisch synthetische Anwendungen genutzt werden kann. Für medizinisch-pharmazeutische Anwendungen ist das Salzcluster einerseits für Tablettierungen geeignet, wobei sich die Tablette beim Pressvorgang mit einem Überzug versieht, der eine Magenpassage ermöglicht. Andererseits ist das Salzcluster auch für die Bereitung von Injektionen und Infusionen geeignet, wobei sowohl eine hypotonische Gabe durch Zugabe von Wasser als auch eine isotonische Gabe durch Zusatz von Natriumbicarbonatlösung oder isotonischer Kochsalzlösung gewählt werden kann.
Thermoanalyse (von 0,609 g = 1/20 d. Ansatzes): 22,2 ml $CO_2$ = 0,99 mMol entsprechend 0,99 mMol Procainiumhydrogencarbonat bei 1/20 der Ansatzgröße werden freigesetzt!
$^1$H-NMR ($D_2O$)[ppm]:7,89, 7,86 (d); 6,86, 6,83 (d); 4,59 (tr); 3,48 (tr), 3,21 (qu); 1,295 (tr)

$^{13}C$-NMR ($D_2O$) [ppm]: 9,093 (2* $CH_3$), 48,583 (2* $CH_{2)}$), 50,817 (1* $CH_2$), 60,14 (1* $CH_2$), 115, 143 (2* aromat. CH), 132,258 (2* arom. CH), 160,781; 153,336 (2* quat. arom. C), 168,655 (OC=O)

MS [m/z]: $ES^+$: 237,7 (236+H); 259,7 (236+Na)

b) Procain / Kohlensäure ($NaHCCO_3$)

236 mg Procain (1 mMol) werden in 30 ml Wasser suspendiert und unter Kühlung auf 0°C Kohlendioxid in die Lösung eingeleitet, bis alles Procain aufgelöst ist. Das Ende der Reaktion, d.h. die Bildung von Procainiumhydrogencarbonat, wird durch Leitfähigkeitsmessungen und Bestimmung der Gefrierpunktsemiedrigung (erhöhte Leitfähigkeit durch Salzbildung, deutliche Gefrierpunktserniedrigung) ermittelt. Da bei der Gefriertrocknung der Procainiumhydrogencarbonat / Kohlensäure-Lösung ein Zerfall des Procainiumhydrogencarbonats in seine Bestandteile Procain, $CO_2$ und Wasser erfolgt, wird in der Kälte eine 4 mMol $NaHCO_3$ enthaltende homogene Lösung hinzugefügt. Die klare Lösung wird eingefroren und anschließend gefnergetrocknet, wobei überschüssiges $CO_2$ im Vakuum abgezogen wird. Es werden 0,65 g (95,6% d.Th.) des Procainiumhydrogencarbonat enthaltenden Salzclusters erhalten.

c) Procain / $H_2SO_4$/ $NaHCO_3$ / $CO_2$

236 mg Procain (1 mMol) werden in 5 ml Wasser suspendiert und unter Kühlung auf 0°C mit 2 ml einer 1n $H_2SO_4$ versetzt. Zu der klaren Lösung werden bei 0°C bis -4° C 5 ml einer gekühlten mit $CO_2$ unter Druck gesättigten und 0,336 g $NaHCO_3$ (4 mMol) enthaltenden homogenen Lösung zugesetzt. Die klare Lösung wird eingefroren und anschließend gefriergetrocknet, bis keine Gewichtsabnahme mehr feststellbar ist. Es werden 0,57 g (94,2% d.Th.) des Procainiumhydrogencarbonat enthaltenden Salzclusters erhalten.

d) Procain / $NaHSO_4$/ $NaHCO_3$ / $CO_2$

236 mg Procain (1 mMol) werden mit 7 ml einer 120,05 mg $NaHSO_4$ enthaltenden wässrigen Lösung versetzt. Zu der klaren Lösung werden bei 0°C bis -4° C 5 ml einer gekühlten mit $CO_2$ unter Druck gesättigten und 0,336 g $NaHCO_3$ (4 mMol) enthaltenden homogenen Lösung zugesetzt. Die klare Lösung wird eingefroren und gefriergetrocknet, bis keine Gewichtsabnahme mehr feststellbar ist. Es werden 0,54 g (91% d.Th.) des Procainiumhydrogencarbonat enthaltenden Salzclusters erhalten.

e) Procain / $CO_2$ / Wasser / $NaHCO_3$ / NaCl

4,72 g Procain (20 mMol) werden unter Kühlung auf ca. 5°C in 100 ml Wasser suspendiert, anschließend mit 5,04 g $NaHCO_3$ (60 mMol) und 1,168 g NaCl ebenfalls unter Kühlung versetzt. Zu der Suspension gibt man in Intervallen von 10 Minuten jeweils Trockeneis in Portionen von 0,25 $cm^3$ unter kräftigem Rühren hinzu. Die Prozedur wiederholt sich so lange, bis alles Procain in Lösung gegangen ist. Die gekühlte klare Lösung wird eingefroren und gefriergetrocknet. Die Gefriertrocknung erfolgt bis zur Gewichtskonstanz, d.h. bis keine Gewichtsabnahme mehr feststellbar ist. Es werden 12 g (98,3% d.Th.) des Procainiumhydrogencarbonat enthaltenden Salzclusters erhalten, Welche direkt für pharmazeutisch-medizinische und chemisch synthetische Anwendungen genutzt werden kann.

f) 49,102 g Procainhydrochlorid werden in 2000 ml mit $CO_2$ gesättigter Kohlensäure gelöst und in der Kälte mit 60,49 g $NaHCO_3$ und 172,6 g NaCl behandelt. Anschließend wird die klare homogene Lösung eingefroren und gefriergetrocknet. Die Gefriertrocknung erfolgt bis zur Gewichtskonstanz, d.h. bis keine Gewichtsabnahme mehr feststellbar ist. Es werden 281,24g (99,63 % d.Th.) des Procainiumhydrogencarbonat enthaltenden Salzclusters erhalten, welches direkt für pharmazeutisch-medizinische Anwendungen, vor allem die Bereitung einer isotonischen Infusionslösung (1,15 g Proccluster in 100 ml Wasser) genutzt werde kann.

**Beispiel 6 Lidocainiumhydrogencarbonat-Salzcluster**

2,705 g Lidocainhydrochlorid (10 mMol) werden in ca. 5 ml Wasser gelöst und bei 0°C bis -4° C mit 40 ml einer gekühlten mit $CO_2$ unter Druck gesättigten und 3,361 g $NaHCO_3$ (40 mMol) enthaltenden homogenen Lösung versetzt. Anschließend wird die klare homogene Lösung allmählich eingefroren, wobei überschüssiges $CO_2$ im Vakuum abgezogen wird. Das Reaktionsgemisch wird gefriergetrocknet, bis keine Gewichtsabnahme mehr feststellbar ist. Es werden 5,65 g (93% d.Th.) des Lidocainiumhydrogencarbonat enthaltenden Salzclusters erhalten.
Thermoanalyse (von 0,607 g = 1/10 d. Ansatzes): 21,2 ml $CO_2$ = 0,95 mMol entsprechend 0,95 mmol Lidocainiumhydrogencarbonat bei 1/10 der Ansatzgröße.
MS [m/z]: $E^+$ 236 (M+H); 258 (M+Na)

**Beispiel 7** N,N-Diethyl,N-(1-hydroxyethyl)-ammonium-hydrogenearbonat-Salzcluster

a) 1,76 g (15,04 mMol) N,N-Diethyl,N-(1-hydroxyethyl)-amin (Diethylaminoethanol) werden mit äqivalenten Mengen verdünnter Salzsäure neutralisiert und bei 0°C bis 4° C mit 100 ml einer mit $CO_2$ gesättigten Kohlensäurelösung versetzt. Anschließend werden 6,721 g (80 mMol) $NaHCO_3$ zugegeben und bis zur völligen Auflösung des Bicarbonats gerührt. Danach wird die klare homogene Lösung eingefroren und gefriergetrocknet. Die Gefriertrocknung erfolgt bis zur Gewichtskonstanz, d.h. bis keine Gewichtsabnahme mehr feststellbar ist. Es werden 8,7 g (96,34 % d.Th.) des N,N-Diethyl,N-(1-hydroxyethyl)-ammonium-hydrogencarbonat enthaltenden Salzclusters erhalten, welches direkt für pharmazeutisch-medizinische und chemische Anwendungen genutzt werden kann. Für medizinisch-pharmazeutische Anwendungen ist das Salzcluster für Tablettierungen geeignet Die Tabletten sind kühl zu lagern, um einer Zersetzung vorzubeugen.

$CO_2$-Freisetzung: aus 0,0501 g Salzcluster werden 1,85 ml $CO_2$ freigesetzt, das entspricht einem Gehalt von 100 % Salzcluster

b) 1,76 g (15,04 mMol) N,N-Diethyl,N-(1-hydroxyethyl)-amin (Diethylaminoethanol) werden bei 0°C bis 4° C mit 100 ml einer mit $CO_2$ gesättigten Kohlensäurelösung versetzt. Anschließend gibt man in Intervallen von 10 Minuten Portionen von ca. 0,25 $cm^3$ Trockeneis hinzu und wiederholt diese Prozedur etwa acht Mal und fügt dann 5,055 g $NaHCO_3$ (60 mMol) und 0,879 g NaCl (15,04 mMol) hinzu und rührt das Reaktionsgemisch bis zur völligen Auflösung der Salze bei ca. 5°C. Danach wird die klare homogene Lösung eingefroren und gefriergetrocknet. Die Gefriertrocknung erfolgt bis zur Gewichtskonstanz, d.h. bis keine Gewichtsabnahme mehr feststellbar ist. Es werden 8,3 g (96,39 % d.Th.) des N,N-Diethyl,N-(1-hydroxyethyl)-ammonium-hydrogencarbonat enthaltenden Salzclusters erhalten, welches direkt für pharmazeutisch-medizinische Zwecke genutzt werden kann. Für medizinisch-pharmazeutische Anwendungen ist das Salzcluster für Tablettierungen geeignet. Die Tabletten sind kühl zu lagern, um einer Zersetzung vorzubeugen.

## Patentansprüche

**1.** Ammoniumsalz/Mineralsalzclathrate mit sauren zweibasischen anionischen Säureresten der allgemeinen Formel I

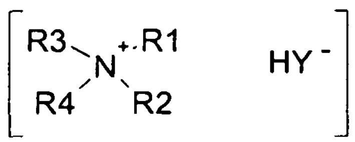

* x Mol Mineralsalz

I

für pharmazeutisch-medizinische und chemisch synthetische Anwendungen, wobei

R1, R2, R3 und R4 = Alkyl und substituiertes Alkyl geradkettig oder verzweigt, gegebenenfalls mit zusätzlicher Alkohol-, Ether-, Silylether-, Ester-, Amino- oder Amid-Funktion, H oder Aryl-alkyl, wobei unter Aryl ein aromatischer oder heteroaromatischer Ring mit gegebenenfalls zusätzlichen Substituenten, wie Alkyl mit 1 bis 4 C-Atomen, OH, $NR^*_2$ mit $R^*_2$= Alkyl mit Alkyl von 1 bis 4 C-Atomen oder H, COOH, COOR, CN, $NO_2$ zu verstehen ist und das kationische positivierte $N^+$ gegebenenfalls Bestandteil eines Wirkstoffes ist,
Y = einen zweibasischen Säurerest einer organischen Dicarbonsäure oder $CO_3^-$, entsprechend $HY^-$ = $HCO_3^-$ und x = 0,5 bis 30 die Zahl der Mineralsalzmoleküle für die Clathratbildung darstellt und
die Ammoniumsalze und Ammoniumsalz / Mineralsalzcluster von Basen als Wirkstoffkomponente abgeleitet sind, wobei unter Wirkstoffbasen Procain, substituierte Procaine, Epinephrin, Tetracain, Lidocain, Bupivacain, Pontocain, Propoxycain, Octacain, Mepivacain, Prilocain, Dibucain, Isocain, Marcain, Etidocain, Piridocain,

Eucain, Butacain, Cocain, Articain, N,N-Diethylaminoethanol, N,N-Dimethylaminoethanol, N-Ethyl, N-methylaminoethanol oder N,N-Diethylaminopropargyl mit freier und geschützter Alkoholfuntion, die verestert, verethert oder silyliert sein kann, zu verstehen sind.

**2.** Verbindung nach Anspruch 1, **dadurch gekennzeichnet, dass** neben der zur Clathratbildung förderlichen Mineralsalze Dextrane, Celluloseester oder Stärke, z.B. Maisstärke, als stabilisierende zur Clathratbildung neigende Stoffe enthalten sind.

**3.** Verbindung gemäß Anspruch 1 oder 2, **dadurch gekennzeichnet, dass** als für die Clathratbildung förderliche Mineralsalze ein-, zwei- und dreiwertige Metallsalzkationen wie $Na^+$, $K^+$, $Li^+$, $Mg^{++}$, $Ca^{++}$, $Zn^{++}$, $Fe^{++}$, $Fe^{+++}$, $Mn^{++}$ und als Anionen $Cl^-$, $Br^-$, $J^-$, $F^-$, $SO_4^-$, $SO_3^-$, $HSO_3^-$, $HCO_3^-$, $PO_4^{3-}$, $HPO_4^-$, $H_2PO_4^-$, $SiO_4^{4-}$, $AlO_2^-$, $SiO_3^-$ und/oder $[(AlO_2)_{12}(SiO_2)_2]^{2-}$ enthalten sind.

**4.** Verfahren zur Herstellung von Verbindungen gemäß den Anspruch 1, 2 oder 3 bei dem mineralsaure Ammoniumsalze, wie $NR_4HSO_4$, $NR_4HSO_3$, $(NR_4)_2HPO_4$, $NR_4H_2PO_4$, $NR_4$Halogenid, mit Halogenid = Cl, Br, I, und / oder organosaure Ammoniumsalze, wie $NR_4$Tosylat, $NR_4$OCO-(Akyl) -COOH, mit $R_4$ in der oben für R1, R2, R3 und R4 angegebenen Bedeutung und mit Alkyl = 0 bis 12 C-Atomen, mit $NaHCO_3$, $NH_4HCO_3$, $Ca(HCO_3)_2$, $Mg(HCO_3)_2$ oder $KHCO_3$ in einem geeigneten Lösungsmittel, gegebenenfalls unter Zusatz von $CO_2$ auch in Form von Trockeneis, unter Druck und für die Stabilisierung erforderlicher Salze, in die entsprechenden Mono-, Di-, Tri-substituierten oder quaternären Ammoniumhydrogencarbonate überführt werden.

**5.** Verfahren zur Herstellung von Verbindungen gemäß Anspruch 4, **dadurch gekennzeichnet, dass** Ammoniumhydrogencarbonat / Mineralsalzclathrate (Einschlussverbindungen, Cluster) der oben angegebenen allgemeinen Formel I, $NR_4HCO_3$ " x Mineralsalz, durch Umsetzung mineralsaurer oder zweibasischer organosaurer Ammoniumsalze in Gegenwart von $Metall^+$ und / oder $Metall^{++}$-hydrogencarbonaten, wie Alkali-oder Erdalkali-Hydrogencarbonaten und / oder Ammoniumhydrogencarbonat unter Zusatz von Kohlensäure ($H_2O$ / $CO_2$), die unter Druck dargestellt wird, und von weiteren Mineralsalzen und / oder Dextranen, Celluloseestern oder Stärke in der Kälte primär zur Umsetzung gebracht und anschließend durch wasserbindende Mittel, z.B. Mineralsalze, oder durch Gefriertrocknung entwässert werden, wobei aufgrund der stabilisierenden Wirkung der Mineralsalze in Form von Clathraten (Einschlussverbindungen, Cluster) auch Dextranen, Celluloseestern oder Stärke diese als beständige lagerungsfähige Feststoffe für pharmazeutisch-medizinische und chemisch synthetische Anwendungen erhalten werden.

**6.** Verfahren zur Herstellung von Verbindungen gemäß Anspruch 5, **dadurch gekennzeichnet, dass** Mono-, Di-, und Tri-Alkylammoniumhydrogencarbonate sich durch Umsetzung der basischen Amine, z.B. Procain, Lidocain oder Diethylaminoethanol, mit Ammoniumhydrogencarbonat ($NH_4HCO_3$) und / oder Kohlensäure, auch unter Zusatz von Trockeneis / Wasser, in der Kälte in situ herstellen lassen, diese aber zwecks Überführung in feste, stabile und lagerungsfähige Salze vor der Entwässerung mit einem Mineralsalz oder Dextran, Celluloseester oder Stärke, z.B. Maisstärke, als stabilisierenden zur Clathratbildung neigenden Stoff, versetzt.

**7.** Verwendung einer Verbindung gemäß einem oder mehreren der Ansprüche 1 bis 3 zur Herstellung pharmazeutisch-medizinischer Produkte.

**8.** Verwendung einer Verbindung gemäß Anspruch 7 zur Herstellung pharmazeutisch-medizinischer Produkte zur Bekämpfung von Schmerzen und entzündlichen Prozessen, gegen Acidose, von Tumorerkrankungen, von Herz-Kreislauferkrankungen, von Autoimmunerkrankungen aufgrund verminderter Wirtsabwehr, zur Rekonvaleszenz und zur "wellness", zur Stressprophylaxe sowie "anti-aging" in der Geriatrie.

**9.** Verwendung einer Verbindung gemäß Anspruch 7, **dadurch gekennzeichnet, dass** diese zur Herstellung pharmazeutischer Formulierungen sowohl in fester Form für orale, dermale, nasale, anale und linguale oder in gelöster Form, auch in Suspension, für parenterale, peritoneale Gabe bzw. für Inhalationen eingesetzt werden, wobei Zusätze weiterer Trägerstoffe, Stabilisatoren, Verdünnungsmitteln und sonstigen Hilfsmitteln, wie sie auf dem Gebiet der Arzneimittel üblich sind, enthalten sein können, und wobei nach Möglichkeit bei der Herstellung unter Ausschluss protischer Lösungsmittel gearbeitet wird, extreme Erwärmung und Feuchtigkeit, außer bei kurzzeitigen Anwendungen, zu vermeiden sind.

**10.** Verwendung gemäß Anspruch 7, **dadurch gekennzeichnet, dass** Carboanhydrasehemmer zusätzlich verwendet werden.

**11.** Verwendung gemäß Anspruch 7, **dadurch gekennzeichnet, dass** die Verbindungen gemäß den Ansprüchen 1-3 in einer Menge von 0,01 mg bis 2000 mg an effektivem Wirkstoff verwendet werden.

## Claims

**1.** Ammonium salt/mineral salt clathrate compounds having an acid dibasic anionic acid residue for pharmaco-medical and chemical synthetic applications of the general formula I,

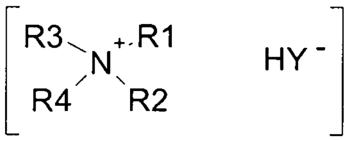 * x mole mineral salt

I

wherein

R1, R2, R3 and R4 = alkyl and substituted alkyl straight-chain or branched, optionally having additional alcohol, ether, silyether, ester, amino or amide function, H or aryl-alkyl, wherein aryl being an aromatic or heteroaromatic ring having optionally additional substituents, such as alkyl having 1 to 4 C atoms, OH, $NR^*_2$ with $R^*_2$= alkyl with alkyl of between 1 and 4 C atoms, or H, COOH, COOR, CN, $NO_2$, and the cationic positive $N^+$ is optionally part of an active agent.
Y = a dibasic acid residue of an organic dicarboxylic acid or $CO_3^-$, corresponding to $HY^- = HCO_3^-$,
and x = 0.5 to 30 represents the number of the mineral salt molecules for clathrate compound formation and the ammonium salts and ammonium salt / mineral salt clathrate compounds are derived from bases as active components, with procaine, substituted procaines, epinephrine, tetracaine, lidocaine, bupivacaine, pontocaine, propoxycaine, octacaine, mepivacaine, prilocaine, dibucaine, isocaine, marcain, etidocaine, piridocaine, eucaine, butacaine, cocaine, articaine, N,N-diethyl aminoethanol, N,N-dimethyl aminoethanol, N-ethyl, N-methyl aminoethanol or N,N-diethyl aminopropargyl with free and protected alcohol function that can be esterified, etherified or silylated, being considered as active agents.

**2.** Compound as set forth in claim 1, wherein apart from the mineral salts that are useful for the clathrate compound formation dextrans, cellulose ester or starch, e.g. cornstarch, are contained as stabilizing substances tending to the formation of clathrate compounds.

**3.** Compound as set forth in claim 1 or 2, wherein monovalent, bivalent and trivalent metal salt cations such as $Na^+$, $K^+$, $Li^+$, $Mg^{++}$, $Ca^{++}$, $Zn^{++}$, $Fe^{++}$, $Fe^{+++}$, $Mn^{++}$ and as anions $Cl^-$, $Br^-$, $J^-$, $F^-$, $SO_4^-$,$SO_3^{--}$, $HSO_3^-$, $HCO_3^-$, $PO_4^{3-}$, $HPO_4^-$, $H_2PO_4^-$, $SiO_4^{4-}$, $AlO_2^-$, $SiO_3^-$ and/or $[(AlO_2)_{12}SiO_2)_2]^{2-}$ are contained as mineral salts that are useful for the formation of clathrate compounds.

**4.** Method for producing compounds as set forth in claim 1, 2 or 3, wherein mineral acid ammonium salts, such as $NR_4HSO_4$, $NR_4HSO_3$, $(NR_4)_2HPO_4$, $NR_4H_2PO_4$, $NR_4$halogen, with halogen = Cl, Br, I, and / or organo-acid ammonium salts, such as $NR_4$Tosylat, $NR_4$OCO-(Akyl) -COOH, with $R_4$ in the above indicated meaning for R1, R2, R3 and R4 and with alkyl = 0 to 12 C atoms, with $NaHCO_3$, $NH_4HCO_3$, $Ca(HCO_3)_2$, $Mg(HCO_3)_2$ or $KHCO_3$ in an appropriate solvent, optionally by adding $CO_2$ even in the form of dry ice under pressure and for the stabilization of required salts, are transformed into the corresponding mono-, di-, tri-substituted or quaternary ammonium bicarbonates.

**5.** Method for producing compounds as set forth in claim 4, wherein ammonium bicarbonate / mineral salt clathrates (inclusion compounds, cluster) of the above mentioned general formula I, $NR_4HCO_3$ * x mineral salt, are primarily brought to transformation in the cold temperature range by transforming mineral acid or dibasic organo-acid ammonium salts in the presence of $metal^+$ and / or $metal^{++}$-bicarbonates, preferably alkali or alkaline earth bicarbonates, and / or ammonium bicarbonate plus the addition of carbon dioxide ($H_2O$ / $CO_2$), which is prepared under pressure, and possibly further mineral salts and / or dextrans, cellulose esters or starch, and afterwards dehydrated by water

binding agents compounds, e.g. mineral salts, or by freeze drying, and due to the stabilizing effect of the mineral salts in form of clathrate compounds (inclusion compounds, cluster), also dextrans, cellulose esters or starch, said compounds are obtained as stable, storable solids for pharmaco-medical and chemical synthetic applications.

**6.** Method for producing compounds as set forth in claim 5, wherein mono-, di- and tri-alkylammonium bicarbonates can be produced in the cold temperature range in situ by the transformation of the basic amines, e.g. procaine, lidocaine or diethyl aminoethanol, with ammonium bicarbonate ($NH_4HCO_3$) and / or carbonic acid, also plus the addition of dry ice / water, but for the transformation into solid, stable and storable salts, a mineral salt or dextran, cellulose ester or starch, e.g. cornstarch, is added as a stabilizing substance tending to the formation of clathrate compounds before the dehydrating process takes place.

**7.** Use of a compound as set forth in one or several of the claims 1 through 3 for the production of pharmaco-medical products.

**8.** Use of a compound as set forth in claim 7 for the production of pharmaco-medical products for fighting against pains and inflammatory processes, against acidosis, tumor diseases, cardiovascular diseases, autoimmune diseases due to a reduced host repulse, for convalescence and "wellness" purposes, for stress prophylaxis and as an "anti-aging" means in geriatrics.

**9.** Use of a compound as set forth in claim 7, wherein said compounds are used for pharmaco-medical applications as well in the solid state for oral, dermal, nasal, anal or lingual applications as in a dissolved state, also in suspension, for parenteral and peritoneal administrations and for inhalations inspectively. For these purposes, further carrier substances, stabilizers, diluting agents and other auxiliary agents that are usual in the field of medicaments, can be contained and, if it is possible, the compounds should be prepared to the exclusion of protic diluting agents and extreme heating and moisture should be avoided, with the exception of short time administrations.

**10.** Use of a compound as set forth in claim 1, that carbonic anhydrase inhibitors can be additionally used.

**11.** Use of a compound as set forth in claim 7, wherein the compounds as said in claim 1-3 are used in doses of 0.01 mg to 2000 mg of the effective active agent.

**Revendications**

**1.** Clathrates du sel d'ammonium/sel minéral avec des résidus acides dibasiques anioniques de la formule générale I

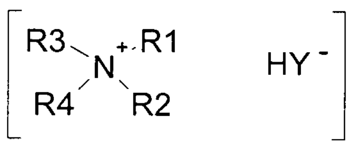 * x mol du sel minéral

I

pour des applications pharmaceutico-médicaux et chimiques de synthèse, dont

R1, R2, R3 und R4 = alkyle et alkyle substitué en chaîne linéaire ou ramifiée, le cas échéant avec fonction supplémentaire d'alcool, éther, éther de silyle, ester, amino ou amide, H ou alkyle d'aryle qui se comprend ici comme un anneau aromatique ou hétéroaromatique avec des substituants additionnels si nécessaire, comme l'alkyle avec 1 à 4 atomes de carbone, OH, NR*2 avec R*2= alkyle avec l'alkyle de 1 à 4 atomes de carbone ou H, COOH, COOR, CN, N02, le cas échéant, le N+ cationique positivé est un composant d'un agent,
Y = un résidu acide dibasique d'un acide dicarboné organique ou $CO_3^-$, correspondant à $HY^- = HCO_3^-$
et x = 0,5 à 30 présent le nombre des molécules du sel minéral pour la clathration et
les sels d'ammonium et les clathrates du sel d'ammonium/sel minéral sont dérivés des bases comme composant d'agent, les bases d'agent étant le procaine, les procaines subtitués, l'épinéphrine, le tetracaine, lidocaine, bupivacaine, pontocaine, propoxycaine, octacain, mepivacaine, prilocaine, dibucaine, isocaine, marcaine, etidocaine, piridocaine, eucaine, butacaine, cocaine, articain, N,N-diéthylaminoéthanol, N,N-diméthylaminoéthanol, N-éthyle, N-méthyl-aminoéthanol ou N,N-diéthylaminopropargyle avec fonction d'alcool libre ou protégée, qui peut être estérifié, éthérifié ou silylé.

**2.** Composé suivant la revendication 1 est **caractérisé en ce que**, à côté des sels minéraux dextrane, ester de cellulose ou amidon p.ex. amidon de maïs qui sont bons pour la clathration, des produits stabilisants, ayant tendance à la clathration sont inclus.

**3.** Composé suivant la revendication 1 ou 2 est **caractérisé en ce que** comme sels minéraux qui sont bons pour la clathration, des cations de sel métallique monovalents, bivalents et trivalents comme $Na^+$, $K^+$, $Li^+$, $Mg^{++}$, $Ca^{++}$, $Zn^{++}$, $Fe^{++}$, $Fe^{+++}$, $Mn^{++}$ et des anions $Cl^-$, $Br^-$, $J^-$, $F^-$, $SO_4^-$, $SO_3^-$, $HSO_3^-$, $HCO_3^-$, $PO_4^{3-}$, $HPO_4^-$, $H_2PO_4^-$, $SiO_4^{4-}$, $AlO_2^-$, $SiO_3^-$ et/ou $[(AlO_2)_{12}(SiO_2)_2]^{2-}$ sont inclus.

**4.** Procédé pour la production des composés suivant la revendication 1, 2 ou 3 pour lequel des sels d'ammonium acides minéraux, comme $NR_4HSO_4$, $NR_4HSO_3$, $(NR_4)_2HPO_4$, $NR_4H_2PO_4$, $NR_4$-halogénure, avec halogénure = Cl, Br, I, et / ou des sels d'ammonium acides organo, comme $NR_4$-tosylate, $NR_4OCO$-(akyle) -COOH, avec $R_4$ dans la signification dénommée ci-dessus pour R1, R2, R3 et R4 et avec l'akyle = 0 á 12 atomes de carbone, avec $NaHCO_3$, $NH_4HCO_3$, $Ca(HCO_3)_2$, $Mg(HCO_3)_2$ ou $KHCO_3$ dans un solvant approprié, le cas échéant en ajoutant de $CO_2$ également sous forme de neige carbonique, sous pression et des sels nécessaires pour la stabilisation, sont transformés en carbonates d'ammonium mono-, di-, trisubstitués ou quaternaires correspondants.

**5.** Procédé pour la production des composés suivant la revendication 4 est **caractérisé en ce que** les clathrates du carbonate d'ammonium / sel minéral (composés d'inclusion, agglomérat de molécules) de la formule générale I dénommée ci-dessus, $NR_4HCO_3$ * x sel minéral, par transformation des sels d'ammonium acides minéraux ou organo dibasiques en présence des hydrogénocarbonates métal$^+$ et / ou hydrogénocarbonates métal$^{++}$, comme hydrogénocarbonates alcalines ou alcalinoterreux et / ou carbonate d'ammonium en ajoutant le dioxyde de carbone ($H_2O$ / $CO_2$), préparé sous pression, et d'autres sels minéraux et / ou dextranes, ester de cellulose ou amidon en premier lieu sont menés à la transformation au froid et sont desséchés après par des substances retenant l'eau, p.ex. des sels minéraux, ou par la lyophilisation, en recevant également des dextranes, esters de cellulose ou amidon comme matières solides stables, qui supportent le stockage, pour des applications pharmaceutico-médicaux et chimiques de synthèse grâce à l'effet stabilisant des sels minéraux sous forme des clathrates (composés d'inclusion, agglomérat de molécules).

**6.** Procédé pour la production des composés suivant la revendication 5 est **caractérisé en ce que** les carbonates d'ammonium de mono-, di- et trialkyle peuvent être produits in situ par la transformation au froid des amines basiques, p.ex. procaine, lidocaine ou diéthylaminoéthanol, avec carbonate d'ammonium ($NH_4HCO_3$) et / ou dioxyde de carbone, également en ajoutant de neige carbonique / eau, mais qui - en vue de la transformation en sels solides et stables, qui supportent le stockage - sont mélangés avant le desséchage avec un sel minéral ou dextrane, ester de cellulose ou amidon p.ex. amidon de maïs comme produits stabilisants, ayant tendance à la clathration.

**7.** Utilisation du composé suivant une ou plusieures des revendications 1 à 3 pour la production des produits pharmaceutico-médicaux.

**8.** Utilisation du composé suivant la revendication 7 pour la production des produits pharmaceutico-médicaux pour la lutte contre les douleurs et processus inflammatoires, contre l'acidose, les maladies tumorales, les maladies cardiovasculaires, des maladies autoimmunes à cause d'une protection d'hôte réduite, pour la convalescence et le « wellness », pour la prophylaxie de stress ainsi que « l'anti-aging » dans le domaine de la gériatrie.

**9.** Utilisation du composé suivant la revendication 7 est **caractérisée en ce qu'**il est utilisé pour la production des formulations pharmaceutiques sous forme solide pour l'application orale, dermique, nasale, anale et linguale ou sous forme dissoute, également en suspension, pour l'application parentérale, péritonéale ou pour les inhalations, dans lesquelles des additifs d'autres porteurs, stabilisateurs, agents de dilution et d'autres agents auxiliaires, comme d'habitude dans le domaine des médicaments, peuvent être inclus et, si possible, dont la production se fait sans l'utilisation des solvants protiques, le chauffage et l'humidité extrêmes sont à éviter sauf s'il s'agit des applications de courte durée.

**10.** Utilisation selon la revendication 7 est **caractérisée en ce que** des inhibiteurs de l'anhydrase carbonique sont utilisés en plus.

11. Utilisation selon la revendication 7 est **caractérisée en ce que** les composés suivant les revendications 1-3 sont utilisés avec une quantité de l'agent effectif de 0,01 mg à 2000 mg.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 9801742 W, Weber, Oettmeler, Reuter
- US 6194456 B1
- US 5149320 A, Dhaliwal, Masih
- US 5209724 A
- SU 878297
- US 5505922 A, Thut, Turner
- GB 349640 A
- EP 0013036 W, Kasch, Goldschmidtt
- US 9305631 W
- US 1907891 A
- US 6194456 B


### In der Beschreibung aufgeführte Nicht-Patentliteratur


- **E.R. Hammerland ; Pederson-Bjergard.** *J. pharm. Sci,* 1961, vol. 50, 24
- **C. Venturello ; R. D. Aloisio.** *Synthesis,* 1985, 33-34